(19) Europäisches Patentamt
European Patent Office
Office européen des brevets

(11) Veröffentlichungsnummer: **0 266 512 B1**

(12) **EUROPÄISCHE PATENTSCHRIFT**

(45) Veröffentlichungstag der Patentschrift: **04.12.91**

(51) Int. Cl.[5]: **C07C 63/70**, C07C 63/00, C07C 229/60

(21) Anmeldenummer: **87113034.0**

(22) Anmeldetag: **07.09.87**

Die Akte enthält technische Angaben, die nach dem Eingang der Anmeldung eingereicht wurden und die nicht in dieser Patentschrift enthalten sind.

# (54) Verfahren zur Herstellung von kernfluorierten Benzoesäure-Derivaten.

(30) Priorität: **19.09.86 DE 3631906**

(43) Veröffentlichungstag der Anmeldung: **11.05.88 Patentblatt 88/19**

(45) Bekanntmachung des Hinweises auf die Patenterteilung: **04.12.91 Patentblatt 91/49**

(84) Benannte Vertragsstaaten: **AT BE CH DE ES FR GB GR IT LI LU NL SE**

(56) Entgegenhaltungen:
**EP-A- 0 164 619**
**EP-A- 0 184 035**
**EP-A- 0 230 947**
**DE-A- 3 142 856**
**DE-A- 3 409 244**

**ORGANIC REACTIONS, Band 5, S. 194-228**

(73) Patentinhaber: **BAYER AG**
**W-5090 Leverkusen 1 Bayerwerk(DE)**

(72) Erfinder: **Petersen, Uwe, Dr.**
**Auf dem Forst 4**
**W-5090 Leverkusen 1(DE)**
Erfinder: **Schriewer, Michael, Dr.**
**Am Thelen Siefen la**
**W-5068 Odenthal(DE)**
Erfinder: **Kysela, Ernst, Dr.**
**Virchowstrasse 14**
**W-5060 Bergisch-Gladbach 1(DE)**
Erfinder: **Grohe, Klaus, Dr.**
**Am Wasserturm 10**
**W-5068 Odenthal(DE)**

**EP 0 266 512 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von Benzoesäure-Derivaten, die wertvolle Zwischenprodukte zur Synthese antibakteriell hochwirksamer Chinoloncarbonsäuren nach dem Cycloaracylierungsverfahren darstellen, wie sie beispielsweise in den folgenden Patentanmeldungen beschrieben werden: Europäische Patentanmeldung 126 355, Deutsche Patentanmeldung 3 420 743, Europäische Patentanmeldungen 183 129, 184 035.

3-Chlor-2,4,5-trifluor-benzoylchlorid kann über eine Chlorierung von 2,4,5-Trifluorbenzoesäure hergestellt werden. Nachteilig bei diesem Verfahren ist, daß hierbei ein Gemisch von kernchlorierten Benzoesäuren entsteht, aus dem nach Aufarbeitung die 3-Chlor-2,4,5-trifluorbenzoesäure durch mehrfaches Umkristallisieren isoliert werden muß. Anschließend kann nach Überführung ins Säurechlorid über eine Chlor-Fluor-Austauschreaktion auch das 3-Chlor-2,4,5-trifluor-benzoylfluorid hergestellt werden (Deutsche Patentanmeldung 3 420 796).

Ein weiteres Verfahren beschreibt die Herstellung von 3-Chlor-2,4,5-trifluorbenzoylchlorid aus 3-Chlor-4-fluor-anilin über ein 11-stufiges Verfahren. Nachteilig ist der umständliche Weg, bei dem einige Schritte überdies nur mit schlechter Ausbeute verlaufen (Europäische Patentanmeldung 183 129). Ein analoges Verfahren betrifft die Herstellung von 3-Brom-2,4,5-trifluor-benzoylchlorid (Europäische Patentanmeldungen 183 129, 184 035). 2,3,4,5-Tetrafluorbenzoesäure wird aus Tetrachlorphthaloylchlorid (Zhurnal Obshchei Khimii 36, 139 [1966]), aus Tetrafluoranthranilsäure (Bull. Chem. Soc. Jap. 45, 2909 [1972]) oder aus Tetrafluorbenzol (Tetrahedron 23, 4719 [1967]; Z. Naturforsch. 31 B, 1667 [1976]) über teilweise aufwendige Schritte hergestellt.

Es wurde jetzt gefunden, daß man die Verbindungen der Formel (I)

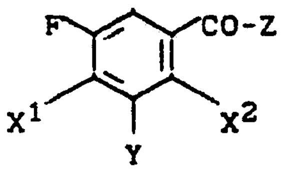

(I),

in welcher

$X^1$ und $X^2$ gleich oder verschieden sind und für Chlor oder Fluor stehen,
Y für Chlor, Brom, Fluor oder Jod und
Z für Hydroxy, Chlor oder Fluor steht,

dadurch erhalten kann, daß man Verbindungen der Formel (II)

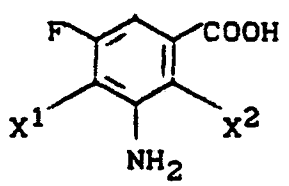

(II),

in welcher $X^1$ und $X^2$ die oben angegebene Bedeutung haben, über eine Sandmeyer- beziehungsweise Balz-Schiemann-Reaktion in Verbindungen der Formel (Ia)

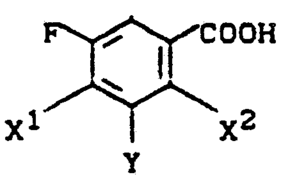

(Ia),

in welcher $X^1$, $X^2$ und Y die oben angegebene Bedeutung haben, überführt und diese Carbonsäuren gegebenenfalls zu einem Säurechlorid der Formel (Ib)

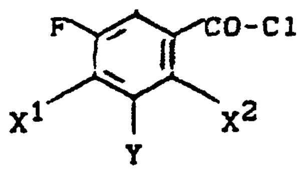

(Ib),

in welcher $X^1$, $X^2$ und Y die oben angegebene Bedeutung haben, umsetzt und dieses in dem Fall, daß mindestens einer der Reste $X^1$ oder $X^2$ für Chlor steht, gegebenenfalls durch eine Fluorierungsreaktion über eine Verbindung der Formel (Ic)

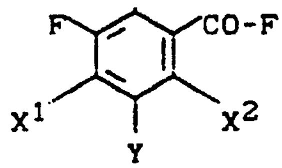

(Ic),

in welcher $X^1$, $X^2$ und Y die oben angegebene Bedeutung haben, zu einer Verbindung der Formel (Id)

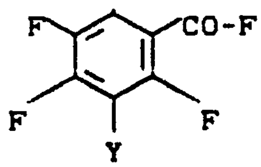

(Id),

in welcher Y die oben angegebene Bedeutung hat, umsetzt,

Alternativ dazu können Verbindungen der Formel (Ie)

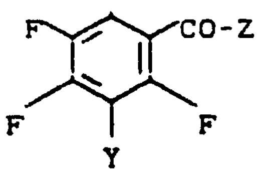

(Ie),

in welcher Y und Z die oben angegebene Bedeutung haben, dadurch erhalten werden, daß man eine Verbindung der Formel (III)

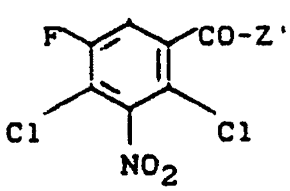

(III),

in welcher Z' Fluor oder Chlor bedeuten kann, in 2,4,5-Trifluor-3-nitro-benzoylfluorid der Formel (IV)

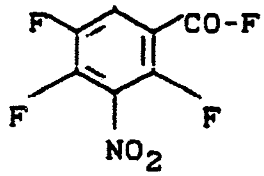

(IV)

überführt, das zur 2,4,5-Trifluor-3-nitro-benzoesäure der Formel (V)

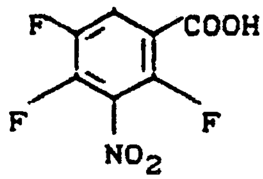

(V)

verseift und danach nach Reduktion der Nitrogruppe über eine Sandmeyer - beziehungsweise Balz-Schiemann-Reaktion in eine Verbindung der Formel (If)

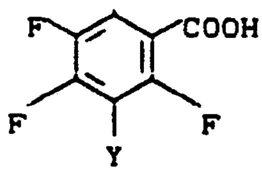

(If),

in welcher Y die oben angegebene Bedeutung hat, umwandelt und diese Carbonsäure der Formel (If) gegebenenfalls in eine Verbindung der Formel (Ig)

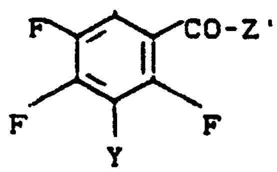

(Ig),

in welcher Y und Z' die oben angegebene Bedeutung haben, überführt.

Der Vorteil des erfindungsgemäßen Verfahrens gegenüber den bekannten Verfahren ist, daß die selektiv verlaufende Umsetzung aus einfach zugänglichen Vorstufen in wenigen Reaktionsschritten zu den gewünschten Zwischenprodukten verläuft. Hierdurch sind die Verbindungen kostengünstiger herstellbar.

Der Reaktionsverlauf des erfindungsgemäßen Verfahrens sei beispielhaft für die Herstellung von 3-Chlor-2,4,5-trifluor-benzoylfluorid aus 2,4-Dichlor-5-fluorbenzoesäure durch das folgende Formelschema beschrieben.

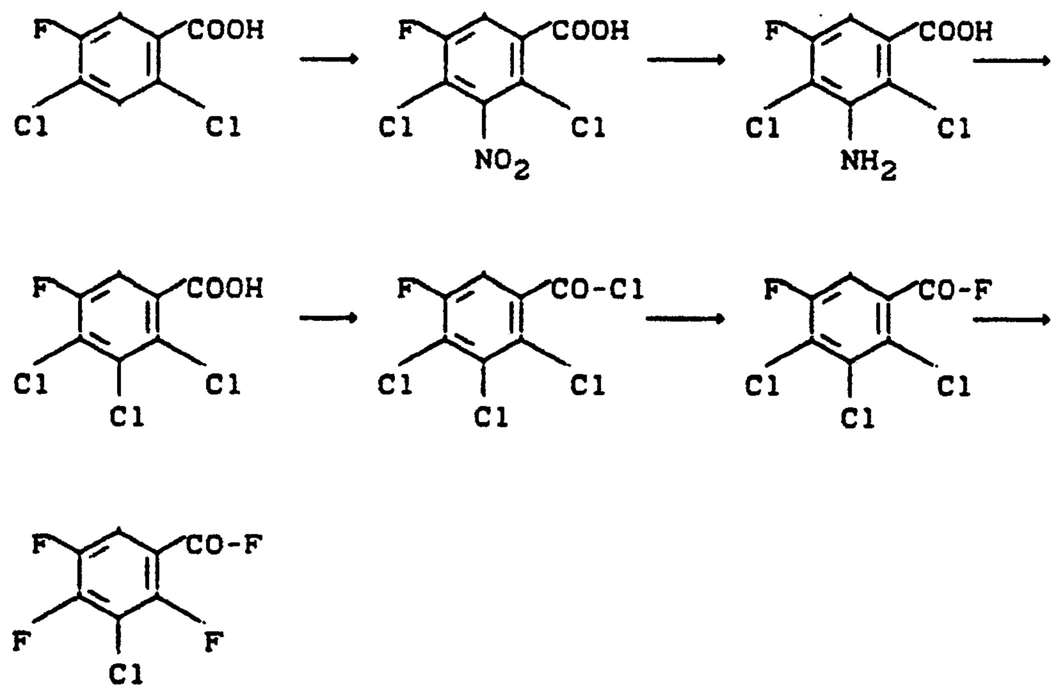

Die als Ausgangsstoffe für das erfindungsgemäße Verfahren verwendeten 3-Amino-2,4-dihalogen-5-fluor-benzoesäuren der Formel (II) sind neu. Man kann sie herstellen, indem man 2,4-Dihalogen-5-fluor-3-nitro-benzoesäuren reduziert. Als Reduktionsmittel kommen beispielsweise Eisen, Zink, Zinn(II)-chlorid, Natriumdithionit, Lithiumaluminiumhydrid oder katalytisch angeregter Wasserstoff in Gegenwart von Katalysatoren wie Raney-Nickel, Raney-Kobalt, Platin oder Palladium in Frage (s. Houben-Weyl, Methoden der Organischen Chemie, Band XI/1, Seite 341 [1957]). Um Enthalogenierung bei der katalytischen Hydrierung zu vermeiden, kann es empfehlenswert sein, die Aktivität des Katalysators durch Zusatz eines Kontaktgiftes, zum Beispiel von Schwefelverbindungen, zu verringern.

Als Lösungsmittel dienen Essigsäure, Essigester, Alkohole wie Methanol, Ethanol, Propanol oder Isopropanol, Glykolmonomethylether, Tetrahydrofuran, Dioxan, Dimethylformamid, Pyridin, Aceton, Wasser oder auch Gemische dieser Lösungsmittel.

Man arbeitet bei Temperaturen von etwa 0 °C bis etwa 120 °C, vorzugsweise von etwa 20 °C bis etwa 80 °C und Normaldruck bei der chemischen Reduktion beziehungsweise bei Temperaturen von etwa 0 °C bis etwa 60 °C und einem Druck von etwa 1 bis 30 bar, vorzugsweise bei Temperaturen von etwa 0 °C bis etwa 30 °C und einem Druck von etwa 1 bis 10 bar bei der katalytischen Reduktion.

Die Überführung der 3-Amino-benzoesäureen (II) beziehungsweise (V) in die Tetrahalogenbenzoesäuren (Ia) beziehungsweise (If) verläuft über eine Sandmeyer-Reaktion, wenn Chlor, Brom oder Jod eingeführt werden sollen (siehe Houben-Weyl, Methoden der Organischen Chemie, Band V/3, Seite 846 [1962], Band V/4, Seiten 438, 641, [1960]) beziehungsweise über eine Balz-Schiemann-Reaktion, wenn Fluor eingeführt werden soll (siehe Houben-Weyl, Methoden der Organischen Chemie, Band V/3, Seite 214 [1962]).

Bei der Sandmeyer-Reaktion wird die 3-Amino-benzoesäure in einer Mineralsäure wie Schwefelsäure, Salzsäure oder Bromwasserstoffsäure durch Zugabe von Natriumnitrit bei Temperaturen von etwa -10 °C bis etwa +10 °C diazotiert und die überschüssige salpetrige Säure durch Zusatz von Amidosulfonsäure oder Harnstoff beseitigt. Die Diazoniumsalz-Lösung wird durch Umsetzung mit einer Lösung von Kupfer(I)-chlorid in konzentrierter Salzsäure, Kupfer(I)-bromid in konzentrierter Bromwasserstoffsäure oder Kaliumiodid in Wasser zur entsprechenden Chlor-, Brom- oder Iodverbindung zersetzt. Man führt die Zersetzung zunächst unter Eiskühlung durch, erwärmt dann auf Raumtemperatur und beendet die Reaktion gegebenenfalls dadurch, daß man bis zum Aufhören der Stickstoffentwicklung die Temperatur bis auf etwa 80 °C steigert.

Bei der Balz-Schiemann-Reaktion wird die Diazoniumsalz-Lösung mit Tetrafluorborsäure oder Natriumtetrafluoroborat in das Diazoniumtetrafluoroborat überführt, das thermisch bei etwa 80-180 °C, vorzugsweise bei etwa 100-150 °C, durch trockene Zersetzung, in Mischung mit Quarzsand oder inerten hochsiedenden Lösungsmitteln wie beispielsweise Chlorbenzol, Toluol, Xylol, Ligroin oder Dioxan zu der entsprechenden 3-Fluorbenzoesäure zersetzt werden kann. Man kann die Diazotierung auch in Fluorwasserstoffsäure bei etwa 0-10 °C vornehmen und anschließend in einem Autoklaven unter Druck bis zur Stickstoffabspaltung auf etwa

50-80 ° C erhitzen oder die thermische Zersetzung auch nach Zusatz geeigneter dipolarer Lösungsmittel wie beispielsweise Dimethylsulfoxid vornehmen.

Die Tetrahalogenbenzoesäuren (Ia) beziehungsweise (If) fallen hierbei als Feststoffe an, die mit einem Chlorierungsmittel wie Thionylchlorid zu den Tetrahalogenbenzoylchloriden und daraus mit Fluorwasserstoff zu den Tetrahalogenbenzoylfluoriden umgesetzt werden können.

Die Herstellung der Verbindungen (Id) aus den Verbindungen (Ib) beziehungsweise der Verbindung (IV) aus der Verbindung (III) kann durch Florierung mit Kaliumfluorid erfolgen.

Die Menge des einzusetzenden Kaliumfluorids richtet sich nach der Anzahl der auszutauschenden Chloratome. Für 1 Chloratom wird mindestens ein Mol Kaliumfluorid eingesetzt, im allgemeinen jedoch 1,1-1,5-Mol. Maximal werden 2 Mol Kaliumfluorid pro 1 Mol Chlor verwendet; darüber hinaus ist die Menge Kaliumfluorid praktisch ohne Einfluß aus den Fluorierungsgrad und das Verfahren wird unökonomisch. Es läßt sich jedoch ein Teil des teuren Kaliumfluorids einsparen, wenn man zuvor das Benzoylchlorid (Ib) mit Fluorwasserstoffsäure fluoriert und das dabei in hoher Ausbeute anfallende Benzoylfluorid (Ic) zur Chlor-Fluor-Austauschreaktion mit Kaliumfluorid einsetzt. Wegen der stärkeren Aktivierung durch die elektronegativere Fluorcarbonylgruppe, ihrer höheren thermischen Stabilität und die verminderte Kaliumchlorid-Belastung im Reaktionsgemisch, führt diese Zweistufenfluorierung insgesamt zu einer verbesserten Bilanz bei der Kernfluorierung. Durch Zusatz eines Katalysators wie 18-Crown-6 kann die Ausbeute noch verbessert werden.

Als Lösungsmittel bei der Kernfluorierung können die für Fluorierungsreaktionen bekannten inerten Lösungsmittel, zum Beispiel Dimethylformamid, Dimethylsulfoxid, N-Methylpyrrolidon, Diethylsulfon und andere verwendet werden. Besonders bevorzugt wird jedoch Tetramethylensulfon (Sulfolan) eingesetzt.

Die Reaktionstemperatur liegt zwischen 160 und 260 ° C in Abhängigkeit vom gewünschten Fluorierungsgrad, vorzugsweise arbeitet man bei Temperaturen von etwa 180 ° C bis etwa 220 ° C.

Die erfindungsgemäß hergestellten Verbindungen sind wertvolle Zwischenprodukte für antibakteriell wirksame Chinoloncarbonsäuren.

Die nachfolgenden Beispiele erläutern die Erfindung.

Beispiel 1

2,3,4-Trichlor-5-fluor-benzoesäure

a) 2,4-Dichlor-5-fluor-3-nitro-benzoesäure

Unter Eiskühlung und Rühren werden 34 ml konz. Schwefelsäure tropfenweise mit 40 ml konz. Salpetersäure versetzt. In dieses Nitriergemisch trägt man portionsweise 20,9 g 2,4-Dichlor-5-fluorbenzoesäure ein, wobei die Temperatur auf 45-50 ° C steigt. Dann wird noch 3 Stunden auf 90-100 ° C erhitzt, das auf Raumtemperatur abgekühlte Gemisch auf 350 ml Eiswasser gegossen, der Niederschlag abgesaugt und mit Wasser gewaschen. Das feuchte Rohprodukt wird in 30 ml Methanol heiß gelöst und die Lösung mit 150 ml $H_2O$ versetzt. Der Niederschlag wird kalt abgesaugt, mit $CH_3OH/H_2O$ gewaschen und im Vakuum bei 80 ° C getrocknet. Es werden 21,2 g rohe 2,4-Dichlor-5-fluor-3-nitro-benzoesäure erhalten. Sie ist genügend rein für die weiteren Umsetzungen. Schmelzpunkt: 192 ° C (aus Toluol/Petrolether).

b) 3-Amino-2,4-dichlor-5-fluor-benzoesäure

254 g (1 mol) 2,4-Dichlor-5-fluor-3-nitro-benzoesäure werden in 1,8 l Ethanol in Gegenwart von 60 g Raney-Nickel bei 11-20 ° C und 10 bar Wasserstoff hydriert, filtriert und im Vakuum eingeengt. Der teigige Rückstand wird mit Wasser verknetet, das auskristallisierte Produkt abgesaugt, mit Wasser gewaschen und getrocknet.

Ausbeute :197 g (88 % der Theorie)
Schmelzpunkt :175-177 ° C, aus Toluol: 184-187 ° C

c) 2,3,4-Trichlor-5-fluor-benzoesäure

A. 86,1 g (0,38 mol) 3-Amino-2,4-dichlor-5-fluor-benzoesäure werden in 1020 ml 50 %iger Schwefelsäure bei etwa 100 ° C gelöst. Die Lösung wird gegebenenfalls über eine Glasfritte filtriert und sogleich unter gutem Rühren im Eisbad gekühlt. Die Diazotierung der ausgefallenen Suspension erfolgt bei -5 ° C bis 0 ° C durch Zutropfen einer Lösung von 33,8 g (0,49 mol) Natriumnitrit in 170 ml Wasser innerhalb 1 Stunde. Anschließend versetzt man portionsweise mit 12,7 g Amidosulfonsäure (schäumt stark) und rührt

15 Minuten nach.

B. Eine Lösung von 16,8 g (0,17 mol) Kupfer(I)-chlorid in 485 ml 17 % Salzsäure wird bei -5° C bis -10° C vorgelegt und die auf -5° C bis 0° C gekühlte Lösung A portionsweise aus einem Scheidetrichter zugegeben. Um den entstehenden Schaum zu zerschlagen, wird intensiv gerührt und bei Bedarf etwas Methylenchlorid zugefügt. Man läßt unter Nachrühren innerhalb etwa 2 Stunden auf Raumtemperatur kommen, saugt ab, wäscht mit Wasser und trocknet bei 70° C im Umluftschrank.

Ausbeute :87 g (94 % der Theorie)
Schmelzpunkt : 157-169° C, nach Umkristallisation aus
Toluol : 166-170° C

Das Produkt wird ohne weitere Reinigung weiter umgesetzt.

Beispiel 2

2,3,4-Trichlor-5-fluor-benzoylchlorid

737 g (3,02 mol) 2,3,4-Trichlor-5-fluor-benzoesäure werden in 1,5 l Thionylchlorid bei Raumtemperatur eingetragen und 16 Stunden unter Rückfluß bis zum Aufhören der Gasentwicklung erhitzt. Überschüssiges Thionylchlorid wird abdestilliert und der Rückstand destilliert.

Ausbeute :694,6 g (87,8 % der Theorie)
Siedepunkt : 100-108° C/0,8-2 mbar
Gehalt : 98,5 %ig.

Beispiel 3

2,3,4-Trichlor-5-fluor-benzoylfluorid

480 g (1,83 mol) 2,3,4-Trifluor-5-fluor-benzoylchlorid werden mit 240 g (12 mol) Fluorwasserstoff bei -5 bis 0° C in einem Rührautoklaven mit Konti-Entspannung vorgelegt, die Apparatur druckdicht verschlossen und stufenweise, entsprechend der HCl-Entwicklung, auf 70° C erwärmt. Der entstehende Chlorwasserstoff wird bei 4-5 bar kontinuierlich entspannt. Nach beendeter HCl-Entwicklung wird gekühlt, der Überdruck entspannt und das Reaktionsgemisch fraktioniert.

Ausbeute: 413 g (92 % der Theorie)
Siedepunkt: 114-116° C/16 mbar, $n_D^{20}$ = 1,5560

Beispiel 4

3-Chlor-2,4,5-trifluor-benzoylfluorid

413 g (1,66 mol) 2,3,4-Trichlor-5-fluor-benzoylfluorid werden mit 392 g (6,76 mol) Kaliumfluorid und 870 ml trockenem Sulfolan 6 Stunden auf 180° C erwärmt. Anschließend wird im Vakuum bis zum Siedepunkt von Sulfolan (~ 145° C/20 mbar) andestilliert und das erhaltene Rohdestillat (362 g) nochmals fraktioniert.

Ausbeute : 287 g (73,8 % der Theorie)
Siedepunkt : 67° C/18 mbar, $n_D^{20}$ = 1,4760
Gehalt : 98,8 %ig.

Beispiel 5

3-Brom-2,4-dichlor-5-fluor-benzoesäure

A. 50 g (0,22 mol) 3-Amino-2,4-dichlor-5-fluor-benzoesäure werden wie in Beispiel 1 c beschrieben in 600 ml 50 %iger Schwefelsäure mit 20 g (0,29 mol) Natriumnitrit in 100 ml Wasser diazotiert.

B. Die Lösung A wird innerhalb etwa 50 Minuten bei -10° C zu einer Lösung aus 15 g (0,1 mol) Kupfer(I)-bromid in 200 ml 48 %iger wäßriger Bromwasserstoffsäure unter gutem Rühren getropft, wobei das Reaktionsprodukt unter Aufschäumen ausfällt. Zur Verringerung der Schaumbildung gibt man 100 ml Methylenchlorid zu und rührt ohne Kühlung nach. Nach 3 Stunden wird gekühlt, der Niederschlag abgesaugt, mit Wasser gewaschen und im Vakuum bei 70° C getrocknet.

Ausbeute : 58,3 g (92 % der Theorie)
Schmelzpunkt : 163-169°; nach Umkristallisation aus

Toluol : 175-178 °C
Das Rohprodukt ist für die Folgereaktion genügend rein.

Beispiel 6

3-Brom-2,4-dichlor-5-fluor-benzoylchlorid

58,3 g (0,2 mol) 3-Brom-2,4-dichlor-5-fluor-benzoesäure werden in 350 ml Thionylchlorid bis zum Aufhören der Gasentwicklung 4 Stunden unter Rückfluß erhitzt. Überschüssiges Thionylchlorid wird abdestilliert und der Rückstand fraktioniert.

Ausbeute : 43,7 g (70 % der Theorie)
Siedepunkt : 108-110 °C/0,9-1,3 mbar
Gehalt : 98,2 %ig.

Beispiel 7

3-Brom-2,4,5-trifluor-benzoylfluorid

20 g (0,0653 mol) 3-Brom-2,4-dichlor-5-fluor-benzoylchlorid, 17 g (0,3 mol) Kaliumfluorid und 50 g Sulfolan werden in Gegenwart katalytischer Mengen (~ 20 mg) 18-Crown-6 22 Stunden auf 150 °C erwärmt. Anschließend wird evakuiert und bis zum Siedepunkt von Sulfolan (~ 145 °C/20 mbar) abdestilliert. Die Reinigung erfolgt durch Redestillation des Rohdestillats (15 g, 86 %ig).

Ausbeute : 12,6 g (75 % der Theorie)
Siedepunkt : 62 °C/2 mbar
Gehalt : 94,4 %ig.

Beispiel 8

2,4-Dichlor-5-fluor-3-iod-benzoesäure

11,2 g 3-Amino-2,4-dichlor-5-fluor-benzoesäure werden in 30 ml halbkonzentrierter Salzsäure durch Zugabe von 20 ml 2,5-molarer Natriumnitrit-Lösung bei 0 °-5 °C diazotiert. Eventuell vorhandenes überschüssiges Nitrit wird durch Zugabe von Harnstoff zerstört. Unter Eiskühlung wird eine wäßrige Lösung von 9,1 g Kaliumiodid zugetropft. Nach beendeter Zugabe wird eine Stunde auf 70 °-80 °C erwärmt. Anschließend kühlt man ab, isoliert den Feststoff und kristallisiert ihn aus Toluol/Petrolether (1:1) um.

Ausbeute : 10,0 g (59,7 % der Theorie)
Schmelzpunkt : 185-186 °C

Beispiel 9

2,4-Dichlor-5-fluor-3-iod-benzoesäurechlorid

10,0 g 2,4-Dichlor-5-fluor-3-iod-benzoesäure und 9,5 ml Thionylchlorid werden solange gekocht, bis kein Gas mehr entweicht. Danach wird restliches Thionylchlorid im Vakuum abgezogen. Es bleiben 10,2 g rohes Säurechlorid zurück, das ohne weitere Reinigung eingesetzt werden kann.

Beispiel 10

Eine Mischung aus 35,3 g (0.1 mol) 2,4-Dichlor-5-fluor-3-iod-benzoylchlorid, 26 g (0,45 mol) trockenem Kaliumfluorid und 20 mg 18-Crown-6 in 67,5 g trockenem Sulfolan wird 15 Stunden auf 90 °C erhitzt. Anschließend wird die Mischung, in das gleiche Volumen Wasser eingerührt, die organische Phase isoliert und destilliert. Man erhält 4 g Produktgemisch vom Siedepunkt 120-130 °C/20 mbar und folgender Zusammensetzung:

5% 2,4,5-Trifluor-3-iod-benzoylfluorid,
20% 4(2)-Chlor-2(4),5-difluor-3-iod-benzoylfluorid,
4% 2,4-Dichlor-5-fluor-3-iod-benzoylchlorid,
2% 4(2)-Chlor-2(4),5-difluor-3-iod-benzoesäure,
67% Sulfolan.

Beispiel 11

2,4,5-Trifluor-3-nitro-benzoylfluorid

a) 2,4-Dichlor-5-fluor-3-nitro-benzoylchlorid

254 g (1 mol) 2,4-Dichlor-5-fluor-3-nitro-benzoesäure werden in 700 ml Thionylchlorid unter Zusatz von 3 Tropfen Dimethylformamid bis zum Aufhören der Gasentwicklung unter Rückfluß erhitzt. Überschüssiges Thionylchlorid wird abdestilliert und der Rückstand im Vakuum fraktioniert.

Ausbeute : 237,1 g (87 % der Theorie)
Siedepunkt : 107-115 °C/0,5-0,7 mbar
Schmelzpunkt : 40-42 °C

b) 2,4,5-Trifluor-3-nitro-benzoylfluorid

27,3 g (0,1 mol) 2,4-Dichlor-5-fluor-3-nitro-benzoylchlorid werden mit 26 g (0,44 mol) trockenem Kaliumfluorid und 20 mg 18-Crown-6 in 68 g trockenem Sulfolan 24 Stunden auf 100 °C erwärmt. Anschließend wird die Rohmischung fraktioniert.

Ausbeute : 5 g (Gehalt: 81 %ig; entsprechend 17 % der Theorie)
Siedepunkt : 80-90 °C / 1 mbar.

Beispiel 12

2,4,5-Trifluor-3-nitro-benzoesäure

3,5 g (0.02mol) 2,4,5-Trifluorbenzoesäure werden in eine Mischung aus 6,8 ml konzentrierter Schwefelsäure und 8 ml 98 %iger Salpetersäure bei 5 - 10 °C eingetragen und ohne Kühlung nachgerührt. Die Temperatur steigt bis auf 70 °C. Man erhitzt 5 Stunden bis auf 80 - 90 °C, gibt nochmals 8 ml 98 %ige Salpetersäure hinzu und erhitzt 2 Stunden auf 80 °C. Danach wird in 100 ml Eiswasser eingetragen, mit Dichlormethan gut extrahiert, mit Natriumsulfat getrocknet und eingeengt. Das zurückbleibende Öl kristallisiert durch. Es wird mit Leichtbenzin verrührt, das Festprodukt abgesaugt und getrocknet.

Ausbeute : 0,5 g, Schmelzpunkt 106 °C
Massenspektrum : m/e 221 ($M^+$), 204 ($M^+$ - OH), 191 ($M^+$ - NO), 158, 130, 30 (NO).

Das entsprechende Amino-Derivat kann nach dem Verfahren gemäß Beispiel 1b) hergestellt werden.

## Patentansprüche

**Patentansprüche für folgende Vertragsstaaten : AT, BE, CH, DE, FR, GB, IT, LI, LU, NL, SE**

**1.** Verfahren zur Herstellung von Verbindungen der Formel (I)

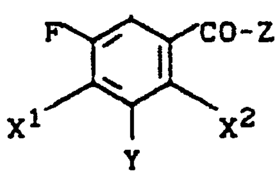

(I),

in welcher

$X^1$ und $X^2$ gleich oder verschieden sind und für Chlor oder Fluor stehen,
Y für Chlor, Brom, Fluor oder Jod und
Z für Hydroxy, Chlor oder Fluor steht,

dadurch gekennzeichnet, daß man Verbindungen der Formel (II)

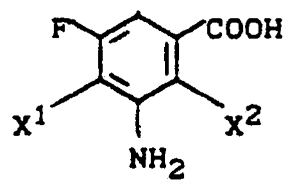

(II),

in welcher $X^1$ und $X^2$ die oben angegebene Bedeutung haben, über eine Sandmeyer- beziehungsweise Balz-Schiemann-Reaktion in Verbindungen der Formel (Ia)

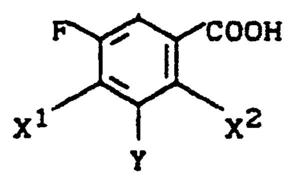

(Ia),

in welcher $X^1$, $X^2$ und Y die oben angegebene Bedeutung haben, überführt und diese Carbonsäuren gegebenenfalls zu einem Säurechlorid der Formel (Ib)

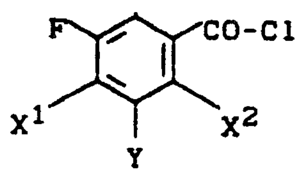

(Ib),

in welcher $X^1$, $X^2$ und Y die oben angegebene Bedeutung haben, umsetzt und dieses in dem Fall, daß mindestens einer der Reste $X^1$ oder $X^2$ für Chlor steht, gegebenenfalls durch eine Fluorierungsreaktion über eine Verbindung der Formel (Ic)

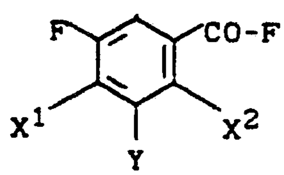

(Ic),

in welcher $X^1$, $X^2$ und Y die oben angegebene Bedeutung haben, zu einer Verbindung der Formel (Id)

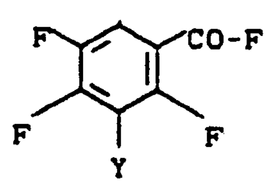

(Id),

in welcher
Y die oben angegebene Bedeutung hat, umsetzt.

2. Verfahren zur Herstellung von Verbindungen der Formel (Ie)

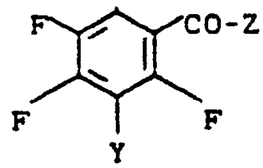

(Ie)

in welcher Y und Z die in Anspruch 1 angegebene Bedeutung haben, dadurch gekennzeichnet, daß man eine Verbindung der Formel (III)

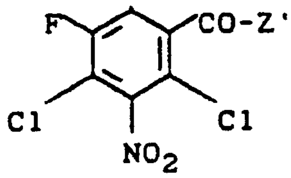

(III)

in welcher Z' Fluor oder Chlor bedeuten kann, in 2,4,5-Trifluor-3-nitro-benzoylfluorid der Formel (IV)

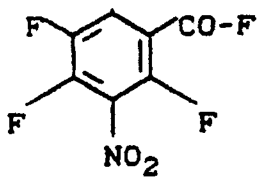

(IV)

überführt, das zur 2,4,5-Trifluor-3-nitro-benzoesäure der Formel (V)

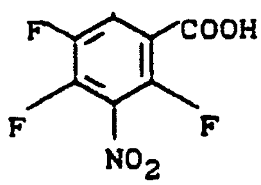

(V)

verseift und danach nach Reduktion der Nitrogruppe über eine Sandmeyer- beziehungsweise Balz-Schiemann-Reaktion in eine Verbindung der Formel (If)

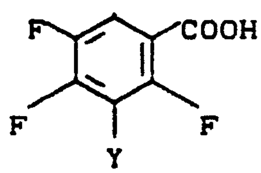

(If),

in welcher Y die oben angegebene Bedeutung hat, umwandelt und diese Carbonsäure der Formel (If) gegebenenfalls in eine Verbindung der Formel (Ig)

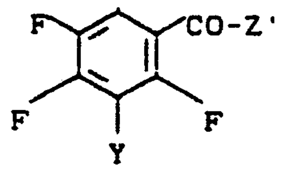

(Ig),

in welcher Y und Z' die oben angegebene Bedeutung haben, überführt.

3. Verbindungen der Formel (II)

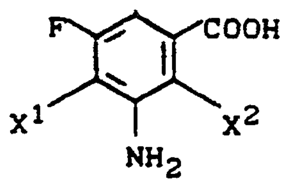

(II),

in welcher

$X^1$ und $X^2$ gleich oder verschieden sind und für Chlor oder Fluor stehen.

**Patentansprüche für folgende Vertragsstaaten : ES, GR**

1. Verfahren zur Herstellung von Verbindungen der Formel (I)

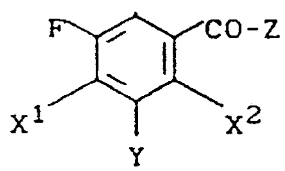

(I),

in welcher

$X^1$ und $X^2$ gleich oder verschieden sind und für Chlor oder Fluor stehen,
Y für Chlor, Brom, Fluor oder Jod und
Z für Hydroxy, Chlor oder Fluor steht,
dadurch gekennzeichnet, daß man Verbindungen der Formel (II)

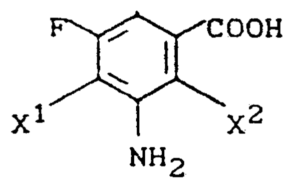

(II),

in welcher $X^1$ und $X^2$ die oben angegebene Bedeutung haben, über eine Sandmeyer- beziehungsweise Balz-Schiemann-Reaktion in Verbindungen der Formel (Ia)

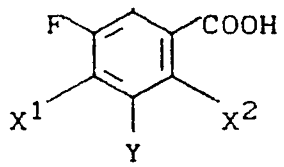

(Ia),

in welcher $X^1$, $X^2$ und Y die oben angegebene Bedeutung haben, über führt und diese Carbonsäuren gegebenenfalls zu einem Säurechlorid der Formel (Ib)

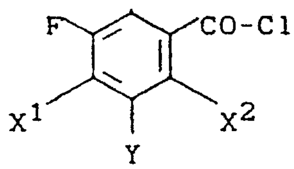

(Ib),

in welcher $X^1$, $X^2$ und Y die oben angegebene Bedeutung haben, umsetzt und dieses in dem Fall, daß mindestens einer der Reste $X^1$ oder $X^2$ für Chlor steht, gegebenenfalls durch eine Fluorierungsreaktion über eine Verbindung der Formel (Ic)

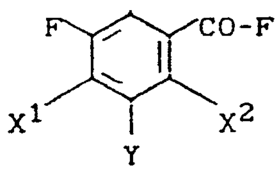

(Ic),

in welcher $X^1$, $X^2$ und Y die oben angegebene Bedeutung haben, zu einer Verbindung der Formel (Id)

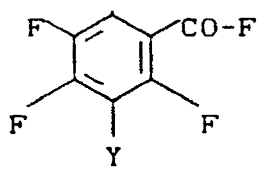

(Id),

in welcher
Y die oben angegebene Bedeutung hat, umsetzt.

2. Verfahren zur Herstellung von Verbindungen der Formel (Ie)

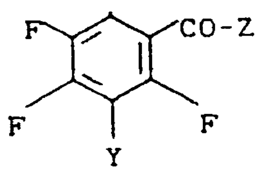

(Ie)

in welcher Y und Z die in Anspruch 1 angegebene Bedeutung haben, dadurch gekennzeichnet, daß man eine Verbindung der Formel (III)

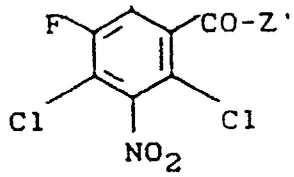

(III)

in welcher Z' Fluor oder Chlor bedeuten kann, in 2,4,5-Trifluor-3-nitro-benzoylfluorid der Formel (IV)

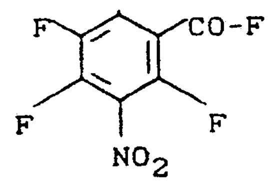

(IV)

überführt, das zur 2,4,5-Trifluor-3-nitro-benzoesäure der Formel (V)

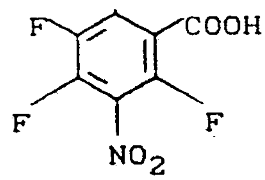

(V)

verseift und danach nach Reduktion der Nitrogruppe über eine Sandmeyer- beziehungsweise Balz-Schiemann-Reaktion in eine Verbindung der Formel (If)

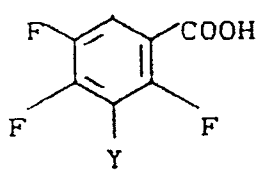

(If),

in welcher Y die oben angegebene Bedeutung hat, umwandelt und diese Carbonsäure der Formel (If) gegebenenfalls in eine Verbindung der Formel (Ig)

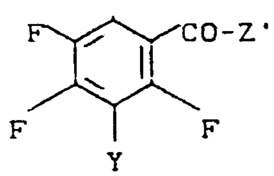

(Ig),

in welcher Y und Z' die oben angegebene Bedeutung haben, überführt.

**Claims**

**Claims for the following Contracting States : AT, BE, CH, DE, FR, GB, IT, LI, LU, NL, SE**

1. Process for the preparation of compounds of the formula (I)

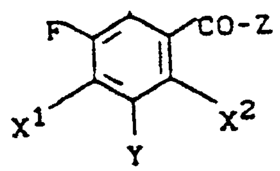

(I),

in which

$X^1$ and $X^2$ are identical or different and represent chlorine or fluorine,

Y represents chlorine, bromine, fluorine or iodine, and

Z represents hydroxyl, chlorine or fluorine,

characterised in that compounds of the formula (II)

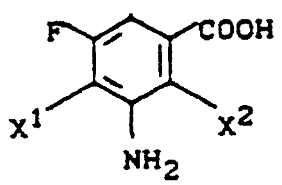

(II),

in which $X^1$ and $X^2$ have the abovementioned meaning, are converted via a Sandmeyer or Balz-Schiemann reaction, into compounds of the formula (Ia)

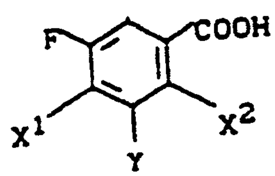

(Ia),

in which $X^1$, $X^2$ and Y have the abovementioned meaning, and, if appropriate, converting these carboxylic acids into an acyl chloride of the formula (Ib)

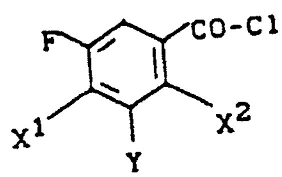

(Ib),

in which $X^1$, $X^2$ and Y have the abovementioned meaning, and, in the case where at least one of the radicals $X^1$ or $X^2$ represents chlorine, converting this, if appropriate through a fluorination reaction, via a compound of the formula (Ic)

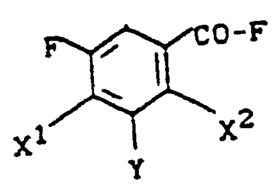

(Ic),

in which $X^1$, $X^2$ and Y have the abovementioned meaning, into a compound of the formula (Id)

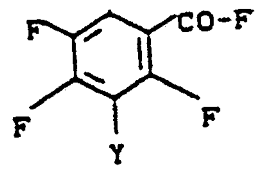

(Id),

in which Y has the abovementioned meaning.

2. Process for the preparation of compounds of the formula (Ie)

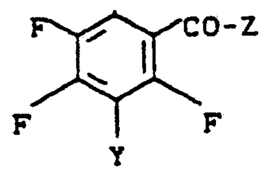

(Ie)

in which Y and Z have the meaning mentioned in Claim 1, characterised in that a compound of the formula (III)

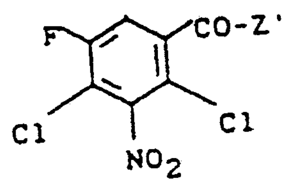

(III)

in which Z' can denote fluorine or chlorine, is converted into 2,4,5-trifluoro-3-nitro-benzoyl fluoride of the formula (IV)

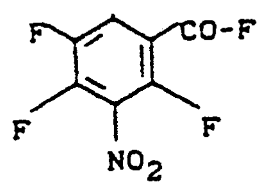

(IV),

which is saponified to form 2,4,5-trifluoro-3-nitro-benzoic acid of the formula (V)

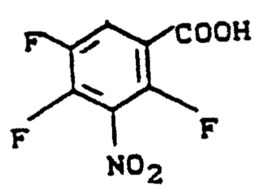

(V)

which, after the nitro group has been reduced, is converted, via a Sandmeyer or Balz-Schiemann reaction, into a compound of the formula (If)

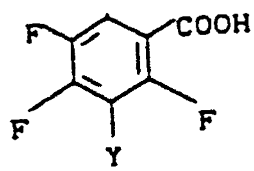

(If),

in which Y has the abovementioned meaning, and, if appropriate, this carboxylic acid of the formula (If) is converted into a compound of the formula (Ig)

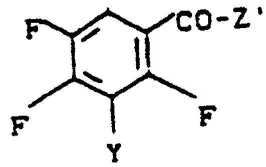

(Ig),

in which Y and Z' have the abovementioned meaning.

**3.** Compounds of the formula (II)

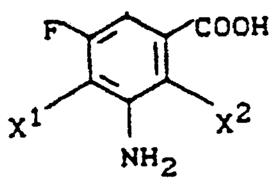

(II),

in which
$X^1$ and $X^2$ are identical or different and represent chlorine or fluorine.

**Claims for the following Contracting States : ES, GR**

**1.** Process for the preparation of compounds of the formula (I)

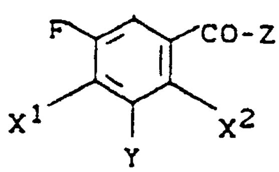

(I),

in which

| | |
|---|---|
| $X^1$ and $X^2$ | are identical or different and represent chlorine or fluorine, |
| Y | represents chlorine, bromine, fluorine or iodine, and |
| Z | represents hydroxyl, chlorine or fluorine, |

characterised in that compounds of the formula (II)

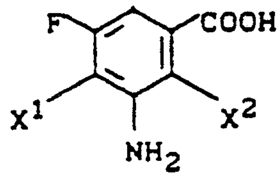

(II),

in which $X^1$ and $X^2$ have the abovementioned meaning, are converted via a Sandmeyer or Balz-Schiemann reaction, into compounds of the formula (Ia)

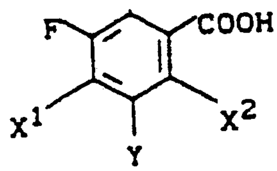

(Ia),

in which $X^1$, $X^2$ and Y have the abovementioned meaning, and, if appropriate, converting these carboxylic acids into an acyl chloride of the formula (Ib)

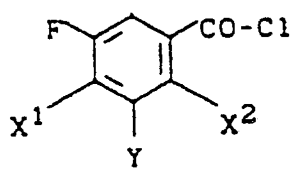

(Ib),

in which $X^1$, $X^2$ and Y have the abovementioned meaning, and, in the case where at least one of the radicals $X^1$ or $x^2$ represents chlorine, converting this, if appropriate through a fluorination reaction, via a compound of the formula (Ic)

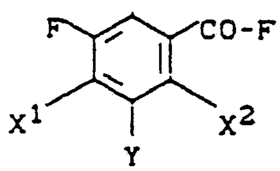

(Ic),

in which $X^1$, $X^2$ and Y have the abovementioned meaning, into a compound of the formula (Id)

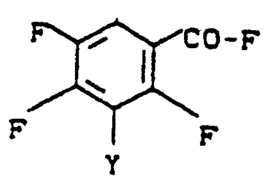

(Id),

in which Y has the abovementioned meaning.

**2.** Process for the preparation of compounds of the formula (Ie)

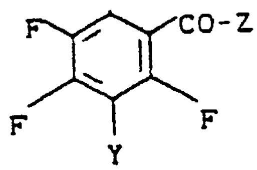

(Ie)

in which Y and Z have the meaning mentioned in Claim 1, characterised in that a compound of the formula (III)

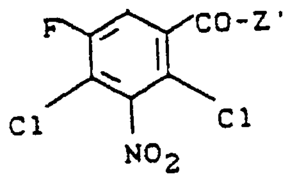

(III)

in which Z' can denote fluorine or chlorine, is converted into 2,4,5-trifluoro-3-nitro-benzoyl fluoride of the formula (IV)

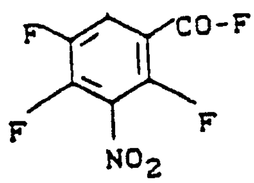

(IV),

which is saponified to form 2,4,5-trifluoro-3-nitro-benzoic acid of the formula (V)

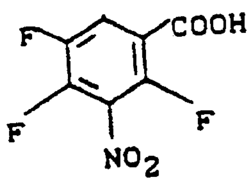

(V)

which, after the nitro group has been reduced, is converted, via a Sandmeyer or Balz-Schiemann reaction, into a compound of the formula (If)

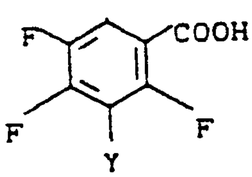

(If),

in which Y has the abovementioned meaning, and, if appropriate, this carboxylic acid of the formula (If) is converted into a compound of the formula (Ig)

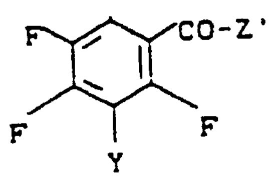

(Ig),

in which Y and Z' have the abovementioned meaning.

**Revendications**

**Revendications pour les Etats contractants suivants : AT, BE, CH, DE, FR, GB, IT, LI, LU, NL, SE**

1. Procédé de préparation de composés de formule

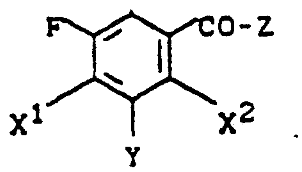

(I),

dans laquelle

$X^1$ et $X^2$, ayant des significations identiques ou différentes, représentent le chlore ou le fluor,

Y représente le chlore, le brome, le fluor ou l'iode, et

Z représente un groupe hydroxy, le chlore ou le fluor,

caractérisé en ce que l'on convertit des composés de formule II

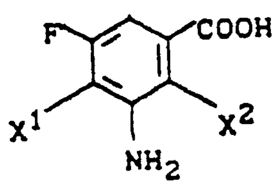

(II),

dans laquelle $X^1$ et $X^2$ ont les significations indiquées ci-dessus, par une réaction de Sandmeyer ou de Balz-Schiemann en composés de formule Ia

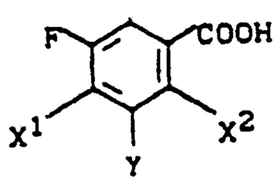

(Ia),

dans laquelle $X^1$, $X^2$ et Y ont les significations indiquées ci-dessus, on convertit éventuellement ces acides carboxyliques en chlorures d'acides de formule Ib

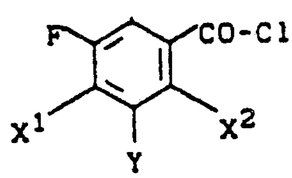

(Ib),

dans laquelle $X^1$, $X^2$ et Y ont les significations indiquées ci-dessus, et on convertit ces derniers éventuellement dans le cas où au moins un des symboles $X^1$ et $X^2$ représente le chlore, en passant, par une réaction de fluoration, par l'intermédiaire d'un composé de formule Ic

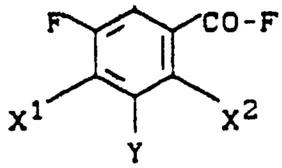

(Ic),

dans laquelle $X^1$, $X^2$ et Y ont les significations indiquées ci-dessus, en composés de formule Id

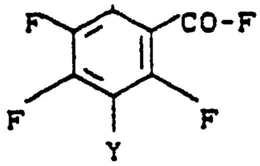

(Id),

dans laquelle Y a les significations indiquées ci-dessus.

**2.** Procédé de préparation des composés de formule Ie

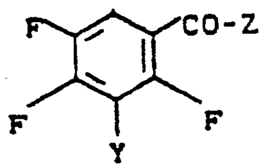

(Ie)

dans laquelle Y et Z ont les significations indiquées dans la revendication 1, caractérisé en ce que l'on convertit un composé de formule III

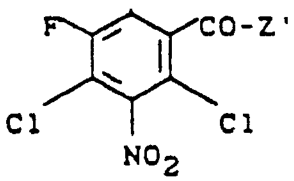

(III)

dans laquelle Z' représente le fluor ou le chlore, en le fluorure de 2,4,5-trifluoro-3-nitrobenzoyle de formule IV

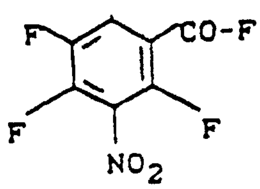

(IV)

qu'on saponifie en l'acide 2,4,5-trifluoro-3-nitrobenzoïque de formule V

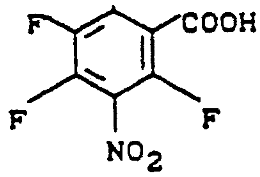

(V)

qu'on convertit ensuite, après réduction du groupe nitro, par une réaction de Sandmeyer ou de Balz-Schiemann, en un composé de formule If

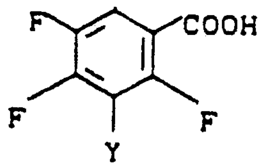

(If),

dans laquelle Y a les significations indiquées ci-dessus,
cet acide carboxylique de formule If étant éventuellement converti en un composé de formule Ig

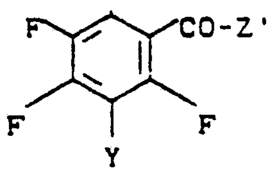

(Ig),

dans laquelle Y et Z' ont les significations indiquées ci-dessus.

**3.** Composés de formule II

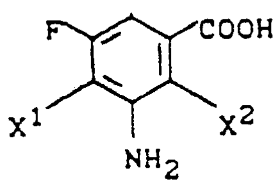

(II),

dans laquelle $X^1$ et $X^2$, ayant des significations identiques ou différentes, représentent le chlore ou le fluor.

**Revendications pour les Etats contractants suivants : ES, GR**

**1.** Procédé de préparation de composés de formule I

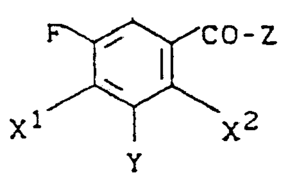

(I),

dans laquelle

$X^1$ et $X^2$, ayant des significations identiques ou différentes, représentent le chlore ou le fluor,
Y représente le chlore, le brome, le fluor ou l'iode, et
Z représente un groupe hydroxy, le chlore ou le fluor,

caractérisé en ce que l'on convertit des composés de formule II

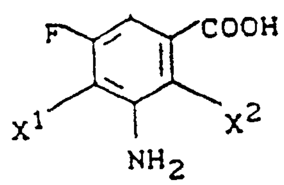
(II),

dans laquelle $X^1$ et $X^2$ ont les significations indiquées ci-dessus, par une réaction de Sandmeyer ou de Balz-Schiemann en composés de formule Ia

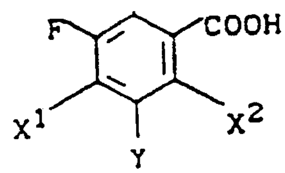
(Ia),

dans laquelle $X^1$, $X^2$ et Y ont les significations indiquées ci-dessus, on convertit éventuellement ces acides carboxyliques en chlorures d'acides de formule Ib

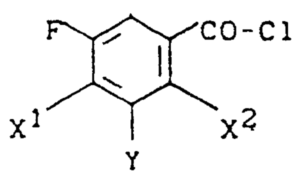
(Ib),

dans laquelle $X^1$, $X^2$ et Y ont les significations indiquées ci-dessus, et on convertit ces derniers éventuellement dans le cas où au moins un des symboles $X^1$ et $X^2$ représente le chlore, en passant, par une réaction de fluoration, par l'intermédiaire d'un composé de formule Ic

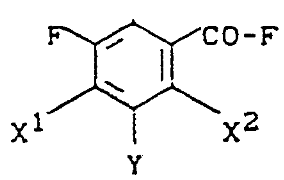
(Ic),

dans laquelle $X^1$, $X^2$ et Y ont les significations indiquées ci-dessus, en composés de formule Id

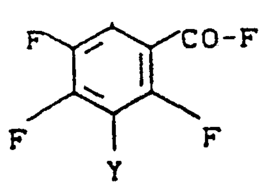
(Id),

dans laquelle Y a les significations indiquées ci-dessus.

**2.** Procédé de préparation des composés de formule Ie

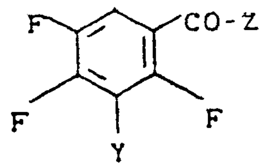

(Ie)

dans laquelle Y et Z ont les significations indiquées dans la revendication 1, caractérisé en ce que l'on convertit un composé de formule III

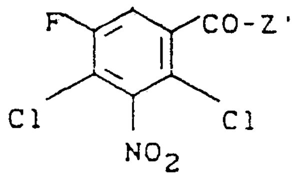

(III)

dans laquelle Z' représente le fluor ou le chlore, en le fluorure de 2,4,5-trifluoro-3-nitrobenzoyle de formule IV

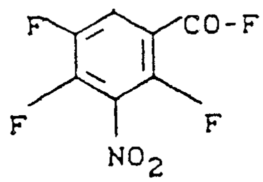

(IV)

qu'on saponifie en l'acide 2,4,5-trifluoro-3-nitrobenzoïque de formule V

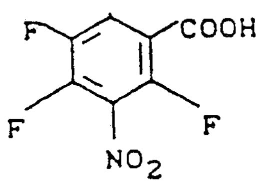

(V)

qu'on convertit ensuite, après réduction du groupe nitro, par une réaction de Sandmeyer ou de Balz-Schiemann, en un composé de formule If

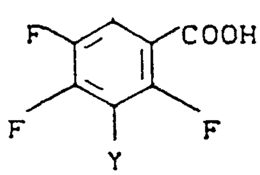

(If),

dans laquelle Y a les significations indiquées ci-dessus,
cet acide carboxylique de formule If étant éventuellement converti en un composé de formule Ig

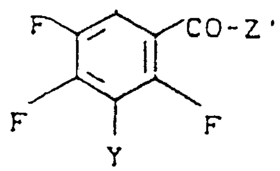

(Ig),

dans laquelle Y et Z' ont les significations indiquées ci-dessus.